# EUROPÄISCHE PATENTANMELDUNG

(11) **EP 4 420 590 A1**
(43) Veröffentlichungstag der Anmeldung: **28.08.2024**
(21) Anmeldenummer: 24159283.1
(22) Anmeldetag: 23.02.2024
(51) Int. Cl.: A61B 1/00, A61B 1/07, A61B 1/267, A61B 1/018

(54) **OPERATIONSLARYNGOSKOP-SPATEL UND DESSEN HERSTELLUNG**

(30) Priorität: 27.02.2023 DE 102023104782
(71) Anmelder: Karl Storz SE & Co. KG, 78532 Tuttlingen (DE)
(72) Erfinder: FANGMANN, Johannes, 78532 Tuttlingen (DE); KÖNIG-URBAN, Kamilla, 78532 Tuttlingen (DE); RIESTER, Fabienne, 78532 Tuttlingen (DE)

(57) **Zusammenfassung**

Ein integral gestalteter Operationslaryngoskop-Spatel (52) aus einem metallischen Grundwerkstoff weist einen Schaft (56) mit einem langgestreckten Grundkörper (60) auf. Der Grundkörper (60) erstreckt sich zwischen einem proximalen Ende (66) und einem distalen Ende (68). Der Grundkörper (60) bildet einen zentralen Hauptkanal (80) und zumindest einen als Lichtleiterkanal nutzbaren Hilfskanal (82) aus. Der Hauptkanal (80) weist beim proximalen Ende (66) ein proximales Endprofil (70) und beim distalen Ende (68) ein distales Endprofil (72) auf. Das proximale Endprofil (70) ist zumindest näherungsweise oval gestaltet. Das distale Endprofil (72) ist geometrisch an den Stimmlippenapparat angepasst. Das proximale Endprofil (70) weist eine Hauptachse (140) auf. Das distale Endprofil (72) weist eine Hauptachse (142) auf. Die Hauptachse (140) des proximalen Endprofils (70) und die Hauptachse (142) des distalen Endprofils (72) sind orthogonal zueinander orientiert. Der zumindest eine Hilfskanal (82) ist in einem proximalen Endbereich (74) benachbart zum proximalen Ende (66) des Grundkörpers (60) vom Hauptkanal (80) beabstandet. Der zumindest eine Hilfskanal (82) mündet in einem distalen Endbereich (76) benachbart zum distalen Ende (68) in den Hauptkanal (80). Ein Verfahren dient zur Herstellung eines solchen Operationslaryngoskop-Spatels (52).

## Beschreibung

Die vorliegende Offenbarung befasst sich mit medizinischen Instrumenten und Bauteilen, insbesondere mit solchen, die zur Bereitstellung eines Zugangs zum Körperinneren genutzt werden, sowie mit deren Herstellung. Insbesondere befasst sich die vorliegende Offenbarung mit der Gestaltung und Herstellung von Spateln für sogenannte Operationslaryngoskope.

Operationslaryngoskope sind medizinische Instrumente, die üblicherweise zur Betrachtung und Behandlung des Kehlkopfes genutzt werden. Eine übliche Anwendung ist die Kehlkopfmikrochirurgie. Operationslaryngoskope erlauben aufgrund einer rohrförmigen Gestaltung des Spatels eine direkte Betrachtung des Kehlkopfes bzw. der Stimmlippen. Operationslaryngoskope stellen üblicherweise weitere Funktionen bereit, umfassend zumindest die Beleuchtung des betrachteten Bereichs (beispielsweise des Kehlkopfes). Der Schaft von Operationslaryngoskopen besteht üblicherweise aus Metallwerkstoffen. Operationslaryngoskope sind im Regelfall keine Einweg-Instrumente. In dieser Hinsicht unterscheiden sich Operationslaryngoskope von Einweg-Laryngoskopen, die beispielsweise in der Notfallmedizin für die Intubation verwendet werden. Einweg-Laryngoskope weisen üblicherweise Spatel auf, die nach der Benutzung weggeworfen werden. Die Spatel von Einweg-Laryngoskopen sind beispielsweise aus Kunststoffen gebildet.

Ein Operationslaryngoskop konventioneller Bauart besteht üblicherweise aus mehreren Bauteilen, die aufwendig bearbeitet und miteinander verbunden werden müssen. Dies umfasst zum einen eine aufwändige subtraktive Bearbeitung und zum anderen relativ aufwändige Fügeprozesse. Es sind verschiedene Arbeitsschritte erforderlich, so dass die Fertigungszeit beträchtlich ist. Häufig sind auch zeitaufwendige manuelle Arbeitsschritte erforderlich. Beispielsweise sind Fügestellen häufig sichtbar, so dass gegebenenfalls eine aufwändige Nachbearbeitung erforderlich ist.

Für die Nutzung als medizinisches Instrument müssen verschiedenste regulatorische Vorschriften erfüllt werden, beispielsweise gilt in der Europäischen Union die sogenannte Medizinprodukteverordnung. Damit steigt mit der Anzahl der verwendeten Bauteile und der verschiedenen Fertigungsschritte der Aufwand für die Dokumentation und Validierung erheblich. Dies gilt umso mehr, wenn manuelle Fertigungsschritte erforderlich sind.

Etablierte Fertigungsprozesse für Spatel für Operationslaryngoskope beinhalten eine große Anzahl an manuellen Prozessschritten, die Auswirkungen auf die Fertigungszeit und die Qualität des Endproduktes haben. Dies ist zum einen durch die Anzahl der erforderlichen Einzelteile bedingt. Hinsichtlich der notwendigen Prozessschritte und Bearbeitungen sind gegebenenfalls Schleifvorgänge, Lötvorgänge und/oder Schweißvorgänge erforderlich. Dies bedingt üblicherweise eine fertigungsgerechte Gestaltung des Instruments, wobei unter Umständen Abstriche hinsichtlich der Anwenderfreundlichkeit, Patientenverträglichkeit und Funktionalität in Kauf genommen werden müssen.

Vor diesem Hintergrund liegt der vorliegenden Offenbarung die Aufgabe zugrunde, einen integral gestalteten Spatel für ein Operationslaryngoskop anzugeben, der hinsichtlich der Anwendung und Patientenverträglichkeit optimiert ist, und der gleichwohl effizient hergestellt werden kann. Insbesondere soll der Spatel möglichst eine optimierte Funktion bereitstellen, die dem medizinischen Fachpersonal und dem Patienten zugutekommt. Dies umfasst gegebenenfalls auch eine anatomisch günstige Gestaltung sowie eine gute Handhabbarkeit. Das Instrument soll sich bei der Anwendung möglichst atraumatisch verhalten.

Die Fertigung soll gemäß einem hierfür geeigneten Verfahren erfolgen, das eine Funktionsintegration und Bauteilintegration ermöglicht. Die Herstellung soll mit möglichst wenigen Schritten erfolgen und eine an den Einsatzzweck angepasste Gestaltung ermöglichen, insbesondere betreffend auch die Reinigung und Aufbereitung des Instruments. Ferner soll der Aufwand für die Herstellung des Instruments (beispielsweise die Stückkosten) verringert werden, insbesondere auch betreffend manuellen Zusatzaufwand. Darüber hinaus soll das Instrument wiederholgenau und prozesssicher herstellbar sein.

Gemäß einem ersten Aspekt bezieht sich die vorliegende Offenbarung auf einen integral gestalteten Operationslaryngoskop-Spatel aus einem metallischen Grundwerkstoff, mit einem Schaft mit einem langgestreckten Grundkörper, der sich zwischen einem proximalen Ende und einem distalen Ende erstreckt und einen zentralen Hauptkanal und zumindest einen als Lichtleiterkanal nutzbaren Hilfskanal ausbildet,
wobei der Hauptkanal beim proximalen Ende ein proximales Endprofil und beim distalen Ende ein distales Endprofil aufweist,
wobei das proximale Endprofil zumindest näherungsweise oval gestaltet ist,
wobei das distale Endprofil geometrisch an den Stimmlippenapparat angepasst ist,
wobei das proximale Endprofil eine Hauptachse aufweist,
wobei das distale Endprofil eine Hauptachse aufweist,
wobei die Hauptachse des proximalen Endprofils und die Hauptachse des distalen Endprofils orthogonal zueinander orientiert sind, und
wobei der zumindest eine Hilfskanal in einem proximalen Endbereich benachbart zum proximalen Ende des Grundkörpers vom Hauptkanal beabstandet ist und in einem distalen Endbereich benachbart zum distalen Ende in den Hauptkanal mündet.

Die Aufgabe der Offenbarung wird auf diese Weise gelöst.Insbesondere handelt es sich bei dem Operationslaryngoskop-Spatel um einen additiv gefertigten Spatel auf Basis eines Metallwerkstoffs. Insbesondere ist der Operationslaryngoskop-Spatel einstückig gestaltet.

Die additive Fertigung (AM - additive manufacturing) erlaubt eine Optimierung des Bauteils insgesamt, wobei dies beispielsweise auf die Integration mehrerer Funktionen sowie auf die Vermeidung von Montageübergängen zurückgeht. In einer beispielhaften Ausgestaltung ist der Spatel integral gestaltet und integral gefertigt.

Der Grundkörper bildet vor allem den Hauptkanal, aber auch den zumindest einen Nebenkanal aus. Der Hauptkanal weist beim proximalen Ende ein erstes spezifisches Profil (proximales Endprofil) und beim distalen Ende ein zweites spezifisches Profil (distales Endprofil) auf. Ferner gibt es zumindest in beispielhaften Ausgestaltungen entlang der Längserstreckung des Hauptkanals ein drittes spezifisches Profil, das einen Übergang zwischen dem proximalen Endprofil und dem distalen Endprofil erlaubt. Das dritte spezifische Profil kann als Übergangsprofil oder Minimalprofil bezeichnet werden. In einer beispielhaften Ausgestaltung ist das dritte spezifische Profil entlang der Längserstreckung das Profil mit der kleinsten Querschnittsfläche ("Taille") des Hauptkanals. Das dritte spezifische Profil ist beispielsweise näherungsweise kreisförmig, so dass ein harmonischer Übergang zwischen dem proximalen Endprofil und dem distalen Endprofil ermöglicht ist

Die integrale Fertigung mittels erlaubt beispielsweise eine Variation der Wandstärke entlang der Längserstreckung des Bauteils, so dass einerseits das Gewicht und der Materialbedarf optimiert werden können und gleichzeitig eine besonders patientensichere atraumatische Gestaltung möglich ist.

Die integrale Fertigung vermeidet Fügeprozesse und die Notwendigkeit der Bereitstellung von Einzelteilen, so dass insgesamt auch die Wirtschaftlichkeit verbessert werden kann. Ferner erhöht sich die Fähigkeit zur Individualisierung. Bauteile können gegebenenfalls gezielt an bestimmte Patienten oder Patientengruppen angepasst werden.

Operationslaryngoskope können beispielsweise in der Hals-Nasen- und Ohrenheilkunde zur Inspektion und Durchführung von Eingriffen am Kehlkopf verwendet werden. Es handelt sich dabei um Mehrweg-Operationslaryngoskope, und damit nicht um Wegwerfteile.

Es wird also im Rahmen der vorliegenden Offenbarung nicht nur vorgeschlagen, das Fertigungsverfahren umzustellen. Zusätzlich geht es auch um eine funktionale Optimierung. Auf diese Weise lassen sich weitere Vorteile erreichen, beispielsweise eine Gewichtsoptimierung sowie die Integration verschiedener Funktionen und Komponenten. Sofern auf aufwendige Montageschritte/Fügeschritte/Bearbeitungsschritte verzichtet werden kann, kann insgesamt die Qualität der Bauteile sowie die Wirtschaftlichkeit bei der Herstellung gesteigert werden.

Die gestalterische Optimierung wird durch die Umstellung des Fertigungsverfahrens ermöglicht. Auf diese Weise lässt sich insgesamt ein Spatel für ein Operationslaryngoskop erzeugen, dessen Querschnitt und dessen Wandstärke über die Längserstreckung variiert sind. Es lassen sich neben dem Hauptkanal Hilfskanäle in günstiger Gestaltung und Orientierung implementieren, so dass sich die Funktionalität verbessert. Dies bezieht sich beispielsweise auf die günstige Anordnung von Lichtquellen bzw. Lichtleitern zur Beleuchtung der beobachteten Stelle im Körper, wobei die Beobachtung üblicherweise durch den Hauptkanal hindurch erfolgt.

Der Spatel wird im Wesentlichen integral durch additive Fertigung erzeugt. Dies erlaubt eine Wandstärkenvariation. Dies kann dazu führen, dass potenziell hochbelastete Abschnitte größere Wandstärken aufweisen als minderbelastete Abschnitte. Ferner kann das Bauteil zur Reduzierung der Verletzungsneigung gezielt lokal aufgedickt werden, um ein möglichst atraumatisches Verhalten zu bewirken.

Gleichwohl kann mit der neuen Gestaltung eine Gewichtsreduktion erzielt werden, einhergehend mit reduziertem Materialbedarf. Die ergibt sich auch durch den wesentliche reduzierten Nachbearbeitungsaufwand. Eine aufwändige Montage von Einzelteilen soll vermieden und somit die Gefahr von Ablagerungen an den Montagestellen verhindert werden.

Der Spatel wird im Wesentlichen durch additive Fertigung erzeugt. Dies schließt jedoch eine etwaige Nachbearbeitung nicht aus. Eine Nachbearbeitung umfasst beispielsweise eine glättende oder lokal abtragende Nachbearbeitung. Ziel der Nacharbeit ist beispielsweise die Erzeugung der gewünschten Oberflächenqualität und gegebenenfalls eine Reduzierung der Porosität.

Im Rahmen der vorliegenden Offenbarung bezieht sich der Begriff distal auf denjenigen Abschnitt/Bereich des Instruments, der vom Benutzer abgewandt ist. Mit anderen Worten ist ein distales Ende des Instruments während der medizinischen Prozedur regelmäßig in das Körperinnere eingeführt, beispielsweise bei Laryngoskopen. Im Rahmen der vorliegenden Offenbarung bezieht sich der Begriff proximal auf einen vom distalen Ende abgewandten Abschnitt/Bereich des Instruments, der dem Benutzer zugewandt ist. Dies kann Gestaltungen umfassen, bei denen während der medizinischen Prozedur das distale Ende im Körper und das proximale Ende außerhalb des Körpers angeordnet ist. Dies ist jedoch nicht einschränkend zu verstehen.

Gemäß einer beispielhaften Ausgestaltung erstreckt sich der Hauptkanal zwischen zwei in den Grundkörper integrierten Hilfskanälen, die sich einander gegenüberliegen und insbesondere entlang einer Längserstreckung des Grundkörpers bezüglich des Hauptkanals zueinander symmetrisch gestaltet sind. Beispielsweise sind die zwei Hilfskanäle symmetrisch bezüglich einer Mittelebene durch den Hauptkanal ausgebildet, wobei die Mittelebene parallel zur Hauptachse des distalen Endprofils ist.

Gemäß einer weiteren beispielhaften Ausgestaltung erstreckt sich eine Längsmittelebene durch den Grundkörper, die parallel zur Hauptachse des proximalen Endprofils ist, wobei die Längsmittelebene den Hauptkanal und die Hilfskanäle schneidet. Üblicherweise gibt es zwei Längsmittelebenen, von denen eine parallel zur Hauptachse des proximalen Endprofils und eine andere parallel zur Hauptachse des distalen Endprofils orientiert ist.

Gemäß einer weiteren beispielhaften Ausgestaltung weist der Grundkörper zumindest beim proximalen Ende oder beim distalen Ende ein geschlossenes Profil auf, vorzugsweise beim proximalen Ende und beim distalen Ende. Gemäß dieser Ausgestaltung hat also der Grundkörper keinen seitlichen Schlitz, durch den Instrumente oder dergleichen eingeführt werden können. Es gibt jeweils beim distalen Endbereich und beim proximalen Endbereich Öffnungen, die im Wesentlichen nach distal bzw. nach proximal ausgerichtet sind. Dazwischen ist der Grundkörper vorzugsweise geschlossen.

Durch den Hauptkanal sind Instrumente einführbar, ferner ist eine direkte Beobachtung möglich. Der zumindest eine Hilfskanal dient beispielsweise zur Aufnahme von Beleuchtungstechnik beziehungsweise zur Aufnahme eines Lichtleiters. Zumindest im proximalen Endbereich folgt der Verlauf des Hilfskanals dem Verlauf des Hauptkanals.

Gemäß einer weiteren beispielhaften Ausgestaltung ist der zumindest eine Hilfskanal beim proximalen Ende vom Hauptkanal separiert, wobei zumindest der eine Hilfskanal beim distalen Endbereich spitzwinklig in den Hauptkanal mündet. Spitzwinklig umfasst beispielsweise einen Winkel (bezogen auf die jeweilige Längserstreckung) von über 5° bis etwa 45°, beispielsweise einen Winkel von kleiner als 30°.

Derartige Gestaltungen sind bei einer "gebauten" Gestaltung des Spatels nur sehr schwer erzielbar. Durch die integrale Gestaltung kann der Übergang (die Mündung) zwischen dem zumindest einen Hilfskanal und dem Hauptkanal optimiert werden. Beispielsweise ist der zumindest eine Hilfskanal derart in Bezug auf den Hauptkanal orientiert, dass während der Anwendung Licht in Richtung auf das Zentrum des distalen Endprofils gerichtet wird. Der Lichtkegel kann günstig ausgerichtet werden.

Gemäß einer weiteren beispielhaften Ausgestaltung verläuft der zumindest eine Hilfskanal zumindest in einem Binnenabschnitt zwischen dem proximalen Endbereich und dem distalen Endbereich geradlinig neben dem Hauptkanal. Auf diese Weise kann der Bauraumbedarf insgesamt klein gehalten werden.

Gemäß einer weiteren beispielhaften Ausgestaltung ist das distale Endprofil des Grundkörpers nach distal aufgeweitet, wobei dies insbesondere eine progressive Aufweitung nach distal umfasst. Die Aufweitung des distalen Endprofils erlaubt einen größeren Blickwinkel, so dass ein größerer Bereich ausgeleuchtet und beobachtet werden kann.

Gemäß bestimmten beispielhaften Ausgestaltungen ist die Aufweitung des Grundkörpers im distalen Endbereich zumindest an ihrer Außenseite progressiv. Mit anderen Worten ist die Steigung der Aufweitung nicht konstant, sondern allmählich zunehmend.

In Richtung auf das distale Ende ist der Grundkörper zumindest näherungsweise trichterförmig aufgeweitet. Dies vereinfacht die Anwendung des Instruments.

Gemäß einer weiteren beispielhaften Ausgestaltung ist zumindest das proximale Endprofil des Grundkörpers nach proximal aufgeweitet, wobei dies insbesondere eine progressive Aufweitung nach proximal umfasst. Daher kann auch das proximale Ende des Spatels insgesamt trichterförmig gestaltet sein.

Gemäß einer weiteren beispielhaften Ausgestaltung weist der Grundkörper zumindest beim distalen Ende eine atraumatische Aufdickung auf, so dass der Grundkörper beim distalen Endprofil eine erhöhte Wandstärke aufweist, Insbesondere weist die atraumatische Aufdickung einen vergrößerten Kantenradius bei einer distalen Stirnfläche auf. Auf diese Weise sinkt die Verletzungsgefahr für den Patienten bei der Anwendung des Instruments. Die atraumatische Aufdickung kann beispielhaft als umlaufende Wulst beim distalen Ende des Spatels gestaltet sein. Die atraumatische Aufdickung ist vorteilhafterweise verrundet, um einen Kontakt scharfkantiger/dünnwandiger Teile mit menschlichem Gewebe zu vermeiden.

Gemäß einer weiteren beispielhaften Ausgestaltung ist der Grundkörper zwischen dem distalen Endprofil und dem proximalen Endprofil tailliert. Dies vereinfacht die Handhabung und Anwendung des Instruments.

Gemäß einer weiteren beispielhaften Ausgestaltung ist das distale Endprofil oval. Insbesondere ist das distale Endprofil eiförmig oder nockenförmig. Das distale Endprofil ist günstigerweise an die anatomische Gestaltung der Stimmlippen angepasst. Beispielhaft ist der Bereich mit einer starken Krümmung (kleiner lokaler Radius) auf der Seite ausgebildet, an der der Handgriff am Spatel montiert wird. Beispielhaft befindet sich in diesem Bereich mit starker Krümmung auch die distale Spitze des Spatels.

Gemäß einer weiteren beispielhaften Ausgestaltung ist eine Nebenachse des proximalen Endprofils größer als die Hauptachse des distalen Endprofils, wobei die Nebenachse des proximalen Endprofils bei Betrachtung entlang einer Längserstreckung des Grundkörpers mit der Hauptachse des distalen Endprofils in einer Ebene liegt. Damit ist der Spatel sowohl beim distalen Ende als auch beim proximalen Ende günstig an die Anatomie des Patienten sowie funktionale Anforderungen angepasst.

Gemäß einer weiteren beispielhaften Ausgestaltung nimmt das distale Endprofil bei einer Projektion auf eine Ebene beim distalen Ende, die normal zur Längserstreckung des Grundkörpers ist, eine Fläche ein, die weniger als ein Drittel einer Fläche einer Projektion des proximalen Endprofils auf eine Ebene beim proximalen Ende umfasst, die normal zur Längserstreckung des Grundkörpers ist. Bei gerader Längserstreckung mit entsprechender Längsachse sind die Projektionsebenen parallel zueinander. Bei (leicht) gekrümmter Längserstreckung sind die Projektionsebenen gegebenenfalls unter einem kleinen Winkel relativ zueinander orientiert.

Gemäß einer weiteren beispielhaften Ausgestaltung wird das distale Endprofil, bei Betrachtung entlang einer Längserstreckung des Grundkörpers, nahezu vollständig vom proximalen Endprofil überdeckt. Dies ist jedoch nicht einschränkend zu verstehen.

Gemäß einer weiteren beispielhaften Ausgestaltung weist der Hauptkanal im Grundkörper bei der inneren Mündung des zumindest einen Hilfskanals einen ovalen oder runden Querschnitt auf, in den der Hilfskanal mündet.

Gemäß einer weiteren beispielhaften Ausgestaltung weisen der Hauptkanal und der zumindest eine Hilfskanal beim proximalen Endbereich Zugangsöffnungen auf, die zumindest abschnittsweise in einer gemeinsamen Ebene liegen. Die gemeinsame Ebene kann gemeinsam additiv gefertigt werden.

Gemäß einer weiteren beispielhaften Ausgestaltung weist der Hauptkanal im Grundkörper eine Umfangswand auf, wobei der zumindest eine Hilfskanal zumindest abschnittsweise zum Hauptkanal benachbart ist und eine Umfangswand aufweist, und wobei sich die Umfangswände des zumindest einen Hilfskanals und des Hauptkanals in einem Überschneidungsbereich zumindest abschnittsweise überschneiden.

Die Überschneidung sorgt dafür, dass insgesamt der Materialverbrauch reduziert wird, ohne dass sich Nachteile bei der Festigkeit ergeben. Eine solche Überschneidung im Sinne eines Ineinandereindringens ist mit konventionellen Ansätzen zur Fertigung solcher Spatel nicht oder nur mit erheblichem Aufwand erreichbar.

Gemäß einer weiteren beispielhaften Ausgestaltung weist der Überschneidungsbereich entlang der Längserstreckung zwischen dem zumindest einen Hilfskanal und dem Hauptkanal eine Dicke auf, die gleich einer Dicke eines benachbarten Abschnitts der Umfangswand des Hauptkanals ist. Mit anderen Worten ist dort die Umfangswand des zumindest einen Hilfskanals in die Umfangswand des Hauptkanals eingedrückt bzw. eingebettet. In einer beispielhaften Ausgestaltung tangiert eine Innenfläche des Hilfskanals im Überschneidungsbereich eine (gedachte) Außenkontur der Umfangswand des Hauptkanals.

Gemäß einer weiteren beispielhaften Ausgestaltung ist ein Übergang zwischen den Umfangswänden des zumindest einen Hilfskanals und des Hauptkanals auskehlungsfrei gestaltet. Auskehlungsfrei bedeutet im Rahmen der vorliegenden Offenbarung, dass keine Spitzkehle vorhanden ist. Stattdessen sind solche Bereiche in günstiger Weise mit Material aufgefüllt. Dies erhöht die Steifigkeit und reduziert die Verschmutzungsneigung. Auch die Gefahr des Einklemmens von Fremdkörpern sinkt. Der Winkel aufeinanderstoßender Flächen ist größer 90°, gegebenenfalls bei infinitesimaler Betrachtung.

Gemäß einem weiteren Aspekt bezieht sich die vorliegende Offenbarung auf ein Operationslaryngoskop mit einem Spatel gemäß zumindest einer der hierin beschriebenen Ausgestaltungen, wobei am Spatel ein Handgriff zur Handhabung des Operationslaryngoskops befestigt ist. Insbesondere ist der Handgriff stumpfwinklig oder rechtwinklig in Bezug auf die Haupterstreckungsrichtung des Spatels orientiert und beim proximalen Endbereich mit dem Grundkörper verbunden. In einer beispielhaften Ausgestaltung ist der Handgriff ein Montageteil. In einer beispielhaften Ausgestaltung ist der Handgriff ein integraler Bestandteil eines integral mittels AM gefertigten Operationslaryngoskops.

Gemäß einem weiteren Aspekt bezieht sich die vorliegende Offenbarung auf ein Verfahren zur Herstellung eines medizinischen Instruments in Form eines integral gestalteten Operationslaryngoskop-Spatels gemäß zumindest einer der hierin beschriebenen Ausgestaltungen, mit den folgenden Schritten:
- Bereitstellung einer Datenverkörperung eines Schafts des Operationslaryngoskop-Spatels
- integrale Fertigung des Schafts in einem additiven Fertigungsverfahren auf Basis eines pulverförmigen metallischen Ausgangsmaterials unter Berücksichtigung der Datenverkörperung, und
- Verbinden des Schafts mit einem Griff am proximalen Ende des Grundkörpers des Schafts.

Das Verfahren eignet sich insbesondere zur Herstellung eines Operationslaryngoskops bzw. dessen Spatel gemäß zumindest einer der zuvor beschriebenen Ausgestaltungen. Die additive Fertigung ermöglicht hohe Gestaltungsfreiheit, so dass der Grundkörper, insbesondere der gesamte Spatel, vollständig oder weitgehend integral/einstückig gestaltet werden können. Trotz integraler Gestaltung lassen sich diverse Kanäle in den Grundkörper einbringen, ferner ist die Erzeugung von internen Mündungen zwischen den Kanälen möglich.

Ferner erlaubt die additive Fertigung glatte Übergänge zwischen verschiedenen Gestaltelementen, wodurch die Reinigung/Aufbereitung weiter vereinfacht wird.

Gemäß einer beispielhaften Ausgestaltung des Verfahrens umfasst der Schritt der additiven Fertigung des Spatels eine Fertigung auf Basis eines pulverförmigen austenitischen, rostfreien Stahlwerkstoffs. Ein derartiger Werkstoff ist für medizinische Anwendungen geeignet. Es gibt also eine gute Verträglichkeit für den Patienten. Ferner eignen sich solche Werkstoffe für die Reinigung/Aufbereitung zwischen verschiedenen Anwendungen. Beispielhaft kann es sich bei dem Werkstoff um einen Edelstahl mit der Werkstoffnummer 1.4404 handeln. Solche Edelstähle weisen eine hohe Korrosionsbeständigkeit auf.

Gemäß einer weiteren beispielhaften Ausgestaltung des Verfahrens umfasst der Schritt der Fertigung die Nutzung einer Pulverbett basierten Vorrichtung zur additiven Fertigung, wobei Pulver in einem Bauraum der Vorrichtung durch energiereiche Strahlung erweicht und gefügt wird. Beispielhaft kann es sich um ein SLM-Verfahren (Selektives Laserschmelzen) handeln. Vorstellbar ist grundsätzlich auch ein SLS-Verfahren (Selektives Lasersintern). Beides sind sogenannte Pulverbett-Verfahren.

Bei dem SLM-Verfahren liegt der Werkstoff pulverförmig vor, wobei aus einer Vorratskammer Material in eine Baukammer übertragen und dort schichtweise abgelegt wird, wobei in der Baukammer eine jeweils um die Dicke einer Schicht absenkbare Plattform vorgesehen ist. Die jeweils oberste Schicht wird mit einem Laserstrahl zumindest teilweise geschmolzen, so dass Schicht für Schicht insgesamt ein festes Bauteil mit der gewünschten Geometrie erzeugt wird. Bereiche, in denen das Pulver nicht geschmolzen wird, werden nach dem Aufbau gereinigt, so dass im Ergebnis das weitgehend oder vollständig fertige Bauteil mit den gewünschten Kavitäten/Kanälen vorliegt.

Gemäß einer weiteren beispielhaften Ausgestaltung des Verfahrens erfolgt die Fertigung des Grundkörpers aufrecht stehend, mit vertikaler Orientierung einer Längsachse in Bezug auf die Bauplattform. Querschnittsbereiche ("Scheiben") des Grundkörpers werden gleichzeitig oder zumindest zeitlich überlappend gefertigt. Auf diese Weise ergeben sich hinreichend homogene Eigenschaften im jeweiligen Querschnittsbereich.

Gemäß einer weiteren beispielhaften Ausgestaltung des Verfahrens umfasst der Schritt der Bereitstellung der Datenverkörperung eine Bereitstellung einer einen erwarteten Verzug des Bauteils bei der Fertigung vorwegnehmenden Datenverkörperung. Mit anderen Worten kann aufgrund von Erfahrungen und/oder Simulationen der Verzug des Bauteils vorhergesagt werden, so dass der Verzug "vorgehalten" werden kann. Wenn nun ein künstlich in entgegengesetzter Richtung verzogenes Bauteil der Fertigung zugrunde gelegt wird und sich der erwartete Verzug einstellt, so ergibt sich im Ergebnis die gewünschte Gestalt.

Eine Datenverkörperung ist ein digitales Abbild der Gestalt des Bauteils. Das digitale Abbild kann um weitere Fertigungsinformationen ergänzt sein. Gemäß einer beispielhaften Ausgestaltung des Verfahrens wird bei der Bereitstellung der den erwarteten Verzug vorwegnehmenden Datenverkörperung ein sich aufgrund inhomogener Materialverteilung bzw. Materialanhäufungen ergebender Verzug berücksichtigt.

Gemäß einer weiteren beispielhaften Ausgestaltung umfasst das Verfahren ferner zumindest einen abtragenden Nachbearbeitungsschritt, der beispielsweise ein Strömungsschleifen zumindest des Hauptkanal oder des zumindest einen Hilfskanals umfasst. Beim Strömungsschleifen wird Schleifmittel durch eine Fluidströmung angetrieben und entlang des Werkstücks bewegt. Auf diese Weise kann in den Kanälen die gewünschte Oberflächenqualität erzeugt werden.

Es versteht sich, dass die vorstehend genannten und die nachstehend noch zu erläuternden Merkmale der Erfindung nicht nur in der jeweils angegebenen Kombination, sondern auch in anderen Kombinationen oder in Alleinstellung verwendbar sind, ohne den Rahmen der vorliegenden Erfindung zu verlassen.

Weitere Merkmale und Vorteile der Erfindung ergeben sich aus der nachfolgenden Beschreibung mehrerer bevorzugter Ausführungsbeispiele unter Bezugnahme auf die Zeichnungen. Es zeigen:
- Fig. 1:: eine perspektivische rückwärtige Ansicht auf ein proximales Ende eines Spatels eines Instruments in Form eines Operationslaryngoskops, in konventioneller Gestaltung;
- Fig. 2:: eine perspektivische rückwärtige Ansicht auf ein proximales Ende eines additiv gefertigten Spatels eines Operationslaryngoskops;
- Fig. 3:: eine hälftig geschnittene Ansicht auf den Spatel gemäß Fig. 3, wobei die Ansichtsebene parallel zur Längserstreckung ist;
- Fig. 4:: eine seitliche Ansicht des Spatels gemäß den Figuren 2 und 3, wobei die Ansichtsorientierung gegenüber der Anordnung von Fig. 3 um 90° gedreht ist;
- Fig. 5:: eine perspektivische rückwärtige Ansicht des Spatels, vom proximalen Ende her, wobei der Spatel hälftig geschnitten ist, zur Veranschaulichung der Gestaltung eines Hauptkanals;
- Fig. 6:: eine rückwärtige Teilansicht des proximalen Endes des Spatels in einer Orientierung, die der Orientierung gemäß Fig. 5 ähnlich ist;
- Fig. 7:: eine hälftige Ansicht eines proximalen Endes des Schafts;
- Fig. 8:: eine hälftige Ansicht eines distalen Endes des Schafts, wobei die Ansichtsorientierungen gemäß Fig. 7 und Fig. 8 einander entgegengesetzt sind;
- Fig. 9:: eine auf Fig. 8 basierende Schnittansicht zur Veranschaulichung eines Querschnitts des Schafts im Bereich einer internen Mündung; und
- Fig. 10:: ein Blockdiagramm zur Veranschaulichung einer Ausgestaltung eines Verfahrens zur Herstellung eines medizinischen Instruments.

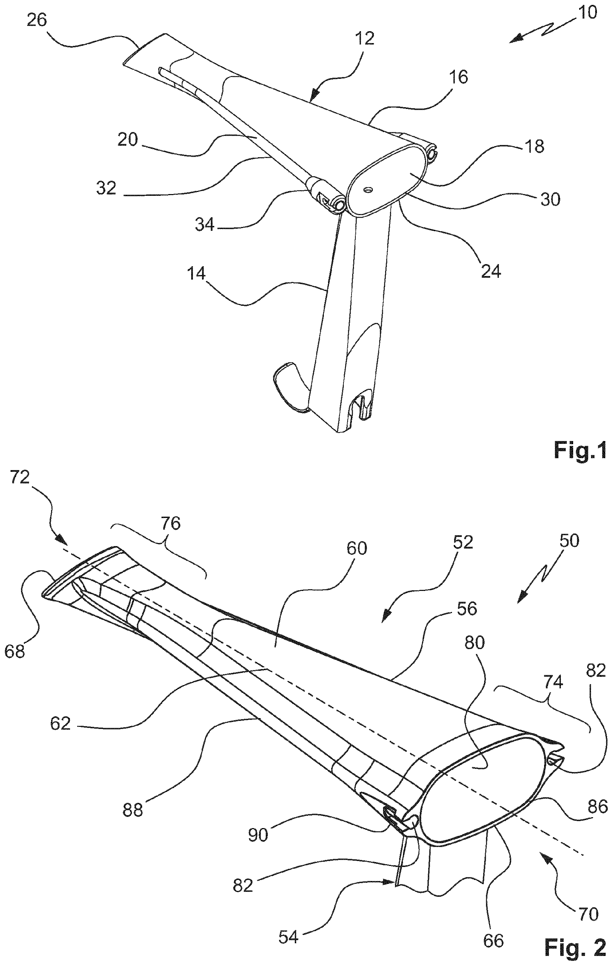

Fig. 1 zeigt anhand einer perspektivischen rückwärtigen Ansicht eine konventionelle Gestaltung eines insgesamt mit 10 bezeichneten Instruments in Form eines Operationslaryngoskops.

Das Operationslaryngoskop 10 umfasst einen Spatel 12, der mit einem Handgriff 14 gefügt ist. Der Spatel 12 bildet einen Schaft 16 aus, der einen Hauptkanal 18 und zumindest einen Hilfskanal 20 umfasst. Üblicherweise sind zwei Hilfskanäle 20 vorgesehen, die beidseits des Hauptkanals 18 ausgebildet sind. Der Schaft 16 erstreckt sich zwischen einem proximalen Ende 24 und einem distalen Ende 26. Der Hauptkanal 18 wird durch ein Rohrstück 30 gebildet. Die Hilfskanäle 20 werden durch separate Bauteile in Form von Rohrstücken 32 und Hülsen 34 gebildet. Es müssen also zur Ausbildung des Spatels 12 zumindest fünf Teile gefertigt und miteinander gefügt werden.

Mit Bezugnahme auf die Figuren 2-9 wird eine offenbarungsgemäße Ausgestaltung eines integral gefertigten Spatels 52 für ein Operationslaryngoskop 50 erläutert. Das Operationslaryngoskop 50 umfasst ferner einen Handgriff 54, der wie zuvor schon beschrieben mit dem Spatel 52 verbindbar oder gegebenenfalls sogar als integraler Bestandteil ausgebildet sein kann.

Der Spatel 52 umfasst einen Grundkörper 60 aus einem Metallwerkstoff. Der Grundkörper 60 weist eine Längserstreckung 62 auf, die Längserstreckung 62 kann streng linear oder leicht gekrümmt sein. Der Grundkörper erstreckt sich zwischen einem proximalen Ende 66 und einem distalen Ende 68. Beim proximalen Ende 66 ist ein proximales Endprofil 70 ausgebildet, das dem Bediener/Operateur zugewandt ist. Beim distalen Ende 68 ist ein distales Endprofil 72 ausgebildet, das dem Patienten zugewandt ist und gegebenenfalls in den Rachen des Patienten eingeführt wird.

Ein proximaler Endbereich des Grundkörpers 60 ist zur Veranschaulichung durch eine mit 74 bezeichnete geschweifte Klammer angedeutet. Ein distaler Bereich ist zur Veranschaulichung durch eine mit 76 bezeichnete geschweifte Klammer angedeutet. Im Grundkörper 60 ist ein Hauptkanal 80 ausgebildet, der sich vom proximalen Ende 66 hin zum distalen Ende 68 erstreckt. Benachbart zum Hauptkanal 80 erstrecken sich im Ausführungsbeispiel zwei Hilfskanäle 82, die an ihrem distalen Ende in den Hauptkanal 80 münden. Um den Hauptkanal 80 erstreckt sich eine Umfangswand 86. Um den jeweiligen Hilfskanal 82 erstreckt sich eine Umfangswand 88. Die Umfangswände 86, 88 durchdringen sich, zumindest abschnittsweise entlang der Längserstreckung 62, gegenseitig. Beim proximalen Ende der Hilfskanäle 82 sind ferner Schnittstellen 90 ausgebildet, die bei der Integration von Lichtleitern oder dergleichen hilfreich sein können.

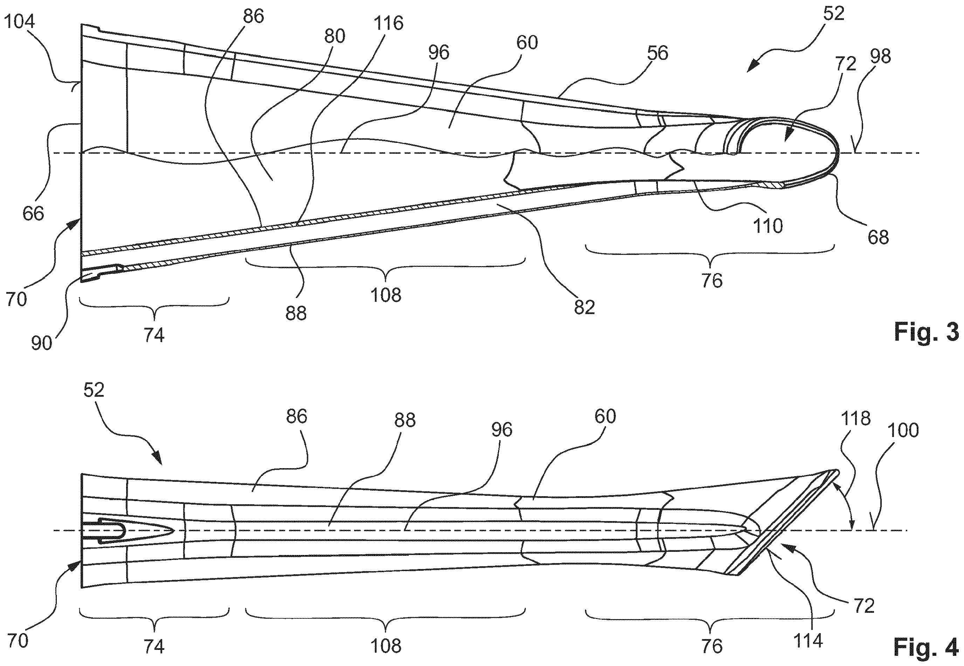

Die Figuren 3 und 4 veranschaulichen jeweils Ansichten, bei denen die Längserstreckung (vergleiche das Bezugszeichen 62 in Fig. 2) des Spatels 52 linear und parallel zur Ansichtsebene ist. Durch den Spatel 52 erstreckt sich jeweils eine Längsachse 96 parallel zur Ansichtsebene. Dies ist nicht einschränkend zu verstehen.

In Fig. 3 ist eine sich ergebende erste Längsmittelebene 98 angedeutet, die senkrecht zur Ansichtsebene und parallel zur Längsachse 96 ist. In Fig. 4 ist eine sich ergebende zweite Längsmittelebene 100 angedeutet, die senkrecht zur Ansichtsebene und parallel zur Längsachse 96 ist. Die beiden Längsmittelebenen 98, 100 sind orthogonal zueinander orientiert.

Beim proximalen Ende 66 des Schafts 52 ist eine Ebene 104 ausgebildet, die durch eine Stirnseite des proximalen Endprofils 70 gebildet ist. Zwischen dem proximalen Endbereich 74 und dem distalen Endbereich 76 ist zu Veranschaulichungszwecken ein Binnenabschnitt 108 angedeutet, der ebenso durch eine geschweifte Klammer gezeigt ist.

Die Ansicht gemäß Fig. 3 ist etwa hälftig geschnitten, so dass der Verlauf des Hauptkanals 80 und der Verlauf des zumindest einen Hilfskanals 82 deutlich wird. Der Hilfskanal 82 mündet bei einer Mündung 110 beim distalen Endbereich 76 in den Hauptkanal. Der Hilfskanal 82 folgt zumindest in einem Binnenabschnitt 108 dem Verlauf des Hauptkanals 80 bzw. von dessen Umfangswand 86. Weil der Hauptkanal 80 sowohl in Richtung auf das proximale Ende 66 als auch in Richtung auf das distale Ende 68 aufgeweitet ist, verläuft die Haupterstreckungsrichtung des Hilfskanals 82 zumindest im Binnenabschnitt 108 spitzwinklig zur Längsachse 96, und nicht parallel dazu.

Die Anordnung des Hilfskanals 82 sowie der Mündung 110 erlauben beispielsweise eine günstige Ausleuchtung einer Beobachtungsebene beim distalen Ende 68 des Instruments, die sich beispielsweise durch das distale Endprofil 82 ergibt.

Zwischen der Umfangswand 86 des Hauptkanals 80 und der Umfangswand 88 des Hilfskanals 82 gibt es einen Überschneidungsbereich 116, in dem sich die Umfangswände 86, 88 überschneiden bzw. ineinander eindringen. Die sich ergebende Wanddicke im Überschneidungsbereich 116 muss jedoch nicht größer als die Dicke der Umfangswand 86 des Hauptkanals 80 sein. Eine weitere Aufdickung dort ist nicht erforderlich. Mit anderen Worten kann die Umfangswand 88 des Hilfskanals 82 dort in die Umfangswand 86 des Hauptkanals 80 eingebettet sein.

Fig. 4 veranschaulicht ferner mit 118 einen Neigungswinkel zwischen einer distalen Endfläche 114 des distalen Endprofils 72 und der Längsmittelebene 100. Auch in dieser Hinsicht ist der Schaft 52 anatomisch günstig gestaltet. Der Neigungswinkel 118 beträgt zumindest im Ausführungsbeispiel etwa 45°. Der Neigungswinkel 118 kann beispielsweise Werte zwischen 30° und 60° annehmen. Die distale Spitze der distalen Endfläche 114 befindet sich auf derjenigen Seite der Längsmittelebene 100, auf der auch der Handgriff (vergleiche Bezugszeichen 54 in Fig. 2) angeordnet/ausgebildet wäre.

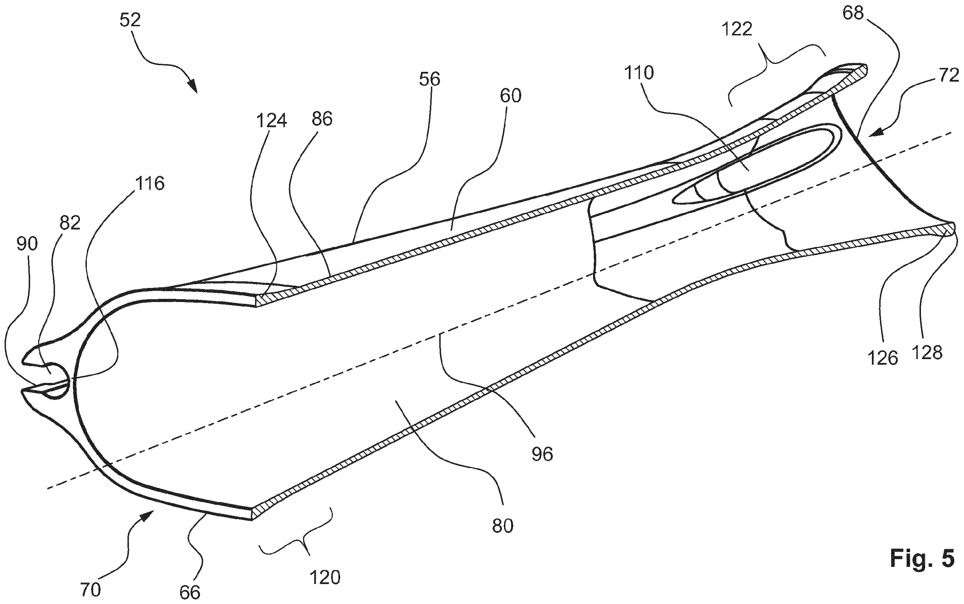

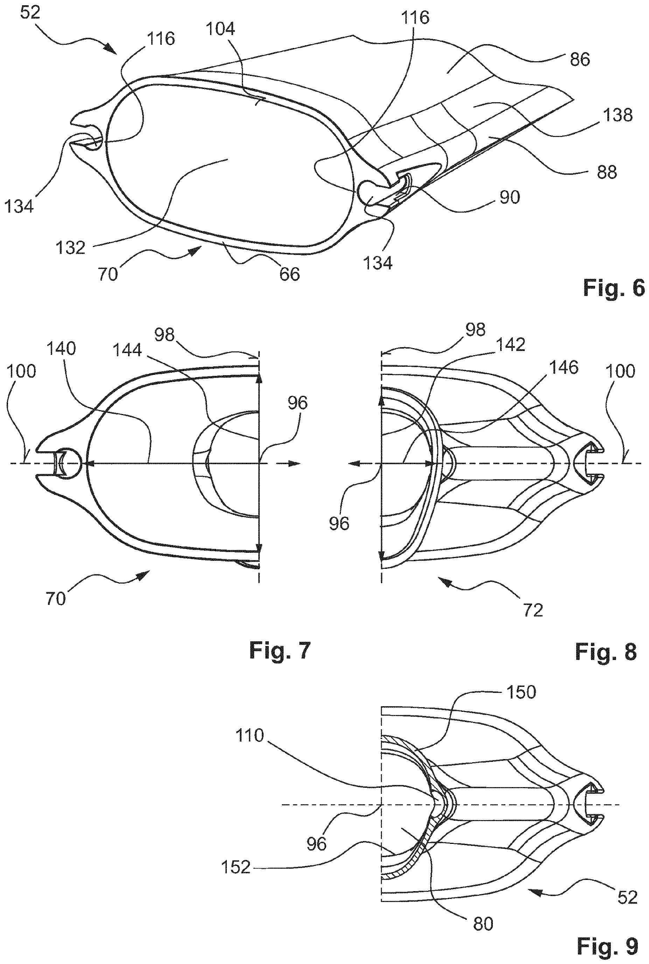

Fig. 5 veranschaulicht anhand einer perspektivischen Darstellung einen Halbschnitt entlang der Längserstreckung des Spatels 52, vom proximalen Ende 66 her. Fig. 6 zeigt eine perspektivische Teilansicht mit ähnlicher Orientierung. Die Schnittebene entspricht der Längsmittelebene 98 in Fig. 3. In Fig. 5 ist die Mündung 110 des Hilfskanals 82 in den Hauptkanal 80 gut sichtbar.

Ferner ergibt sich aus Fig. 5, dass der Hauptkanal 80 beim proximalen Ende 66 eine Aufweitung 120 und beim distalen Ende 68 eine Aufweitung 122 aufweist. Beim proximalen Ende 66 gibt es eine progressive Aufdickung 124. Beim distalen Ende 68 gibt es eine progressive Aufdickung 126, die einen Bereich erhöhter Wandstärke sowie nach distal einen vergrößerten Kantenradius 128 aufweist. Insbesondere die Aufdickung 126 mit dem Radius 128 können das atraumatisch Verhalten des Spatels 52 bei der Anwendung verbessern.

Fig. 6 veranschaulicht insbesondere das proximale Endprofil 70 in der Ebene 104 beim proximalen Ende 66 des Spatels 52. In der Ebene 104 sind Zugangsöffnungen 132, 134 ausgebildet. Die Zugangsöffnung 132 ist dem Hauptkanal 80 zugeordnet. Die Zugangsöffnungen 134 sind den Hilfskanälen 82 zugeordnet. Zwischen der Umfangswand 86 des Hauptkanals 80 und der Umfangswand 88 des Hilfskanals 82 ist ein auskehlungsfreier Übergang 138 ausgebildet. Dort gibt es keinen tief und spitz gestalteten Übergang (im Sinne einer Auskehlung), stattdessen wird ein sanfter Übergang 138 bereitgestellt. Dies verringert die Verschmutzungsneigung.

Die Figuren 7 und 8 veranschaulichen vergleichend die Geometrie des proximalen Endprofils 70 (Fig. 7) und des distalen Endprofils 72 (Fig. 8). Fig. 7 zeigt eine Halbansicht vom proximalen Ende her. Fig. 8 zeigt eine Halbansicht vom distalen Ende her. Die Längsachse 96 ist jeweils senkrecht zur Ansichtsebene. Das proximale Endprofil 70 weist eine Hauptachse 140 und eine Nebenachse 144 auf. Das proximale Endprofil 70 ist insgesamt oval bzw. elliptisch gestaltet. Die Hauptachse 140 ist größer als die Nebenachse 144. Die Hauptachse 140 liegt im Ausführungsbeispiel in der (zweiten) Längsmittelebene 100. Die Nebenachse 144 liegt im Ausführungsbeispiel in der (ersten) Längsmittelebene 98.

Das distale Endprofil 72 ist ebenso etwa oval gestaltet. Das distale Endprofil 72 weist beispielhaft eine Nockenform oder Ei-Form auf, wobei ein spitzeres Ende eines ovalen Längsschnitts des Ovals in der Ansichtsorientierung gemäß Fig. 8 nach unten zeigt. Es ist eine Hauptachse 142 vorhanden, die die größte Erstreckung des distalen Endprofils 72 kennzeichnet. Die Hauptachse 142 liegt im Ausführungsbeispiel in der ersten Längsmittelebene 98. Die Nebenachse 146 liegt im Ausführungsbeispiel in der zweiten Längsmittelebene 98 und kennzeichnet eine (kleinere) Erstreckung des distalen Endprofils 72 senkrecht zur Hauptachse 142. Damit ist das distale Endprofil 72 geometrisch gut an die Stimmlippen angepasst, so dass eine Beobachtung und Behandlung erleichtert wird.

Fig. 9 veranschaulicht einen Schnitt durch den Schaft 52 etwa im Bereich der Mündung 110 (vergleiche auch Fig. 5), wobei die Betrachtung von distal erfolgt, ähnlich wie bei Fig. 8. In diesem Bereich weist der Hauptkanal 80 einen näherungsweise runden oder ovalen Querschnitt 150 auf. Ferner zeigt Fig. 9 anhand des Bezugszeichens 152 ein Minimalprofil, das sich bei einer Engstelle des Hauptkanals 80 entlang der Längserstreckung 62 (vergleiche Fig. 2) bzw. der Längsachse 96 (vergleiche Fig. 5) ergibt. Ausgehend vom Minimalprofil 152 ist der Hauptkanal 80 nach proximal und nach distal aufgeweitet.

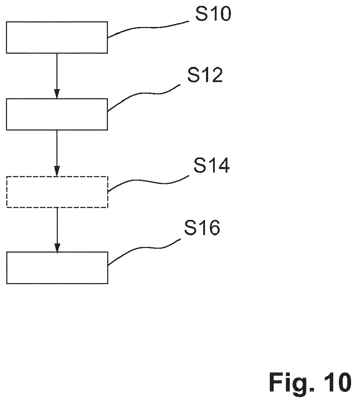

Mit Bezugnahme auf Fig. 10 wird anhand eines Blockdiagramms eine beispielhafte Ausgestaltung eines Verfahrens zur Herstellung eines Bauteils für ein medizinisches Instrument, insbesondere eines schaftförmigen Spatels mit mehreren Kanälen, veranschaulicht. Das Verfahren umfasst einen Schritt S10, der die Bereitstellung einer (CAD-) Datenverkörperung umfasst. Die Datenverkörperung kann auf Basis eines CAD-Modells generiert werden. Die Datenverkörperung wird mittelbar oder unmittelbar einer Anlage zur additiven Fertigung zugeführt, um dort auf dieser Basis den den Schaft bildenden Grundkörper einstückig und integral auszubilden, Schritt S12. Insbesondere erfolgt die additive Fertigung unter Verarbeitung eines Metallwerkstoffes, beispielsweise eines Edelstahlpulvers. Der Grundkörper umfasst zumindest einen ersten Kanal, einen zweiten Kanal und zumindest einen Nebenkanal, der dem zweiten Kanal benachbart und mit diesem verbunden ist. Vorzugsweise wird der Grundkörper auf diese Weise nachbearbeitungsarm oder sogar nachbearbeitungsfrei erzeugt.

Es kann sich ein optionaler Schritt S14 anschließen. Der Schritt S14 umfasst eine Nachbearbeitung, insbesondere eine Oberflächenbearbeitung zumindest eines der Kanäle mittels Strahlschleifen. Auf diese Weise kann etwa im zweiten Kanal oder im ersten Kanal eine gewünschte Oberflächenqualität erzeugt werden.

Schließlich folgt ein Schritt S16, der ein Fügen des Schaftes mit einem Griff umfasst. Dies kann unter Zwischenschaltung eines Verbindungsstückes erfolgen. Auf diese Weise kann insgesamt mit wenigen Schritten ein Instrument mit komplex gestaltetem Schaft hergestellt werden. Dies kann etwa Instrumente in Form von Operationslaryngoskopen Beziehung weise deren Spatel betreffen.

## Patentansprüche

1. Integral gestalteter Operationslaryngoskop-Spatel (52) aus einem metallischen Grundwerkstoff, mit einem Schaft (56) mit einem langgestreckten Grundkörper (60), der sich zwischen einem proximalen Ende (66) und einem distalen Ende (68) erstreckt und einen zentralen Hauptkanal (80) und zumindest einen als Lichtleiterkanal nutzbaren Hilfskanal (82) ausbildet,
wobei der Hauptkanal (80) beim proximalen Ende (66) ein proximales Endprofil (70) und beim distalen Ende (68) ein distales Endprofil (72) aufweist,
wobei das proximale Endprofil (70) zumindest näherungsweise oval gestaltet ist,
wobei das distale Endprofil (72) geometrisch an den Stimmlippenapparat angepasst ist,
wobei das proximale Endprofil (70) eine Hauptachse (140) aufweist,
wobei das distale Endprofil (72) eine Hauptachse (142) aufweist,
wobei die Hauptachse (140) des proximalen Endprofils (70) und die Hauptachse (142) des distalen Endprofils (72) orthogonal zueinander orientiert sind, und
wobei der zumindest eine Hilfskanal (82) in einem proximalen Endbereich (74) benachbart zum proximalen Ende (66) des Grundkörpers (60) vom Hauptkanal (80) beabstandet ist und in einem distalen Endbereich (76) benachbart zum distalen Ende (68) in den Hauptkanal (80) mündet.

2. Spatel (52) nach Anspruch 1, wobei sich der Hauptkanal (80) zwischen zwei in den Grundkörper (60) integrierten Hilfskanälen (82) erstreckt, die sich einander gegenüberliegen und insbesondere entlang einer Längserstreckung (62) des Grundkörpers (60) bezüglich des Hauptkanals (80) zueinander symmetrisch gestaltet sind.

3. Spatel (52) nach Anspruch 2, wobei sich durch den Grundkörper (60) eine Längsmittelebene (100) erstreckt, die parallel zur Hauptachse (140) des proximalen Endprofils (70) ist, und wobei die Längsmittelebene den Hauptkanal (80) und die Hilfskanäle (82) schneidet.

4. Spatel (52) nach einem der Ansprüche 1-3, wobei der Grundkörper (60) zumindest beim proximalen Ende (66) oder beim distalen Ende (68) ein geschlossenes Profil aufweist, vorzugsweise beim proximalen Ende (66) und beim distalen Ende (68).

5. Spatel (52) nach einem der Ansprüche 1-4, wobei der zumindest eine Hilfskanal (82) beim proximalen Ende (66) vom Hauptkanal (80) separiert ist und beim distalen Endbereich (76) spitzwinklig in den Hauptkanal (80) mündet.

6. Spatel (52) nach einem der Ansprüche 1-5, wobei der zumindest eine Hilfskanal (82) zumindest in einem Binnenabschnitt (108) zwischen dem proximalen Endbereich (74) und dem distalen Endbereich (76) geradlinig neben dem Hauptkanal (80) verläuft.

7. Spatel (52) nach einem der Ansprüche 1-6, wobei das distale Endprofil (72) des Grundkörpers (60) nach distal aufgeweitet ist, insbesondere umfassend eine progressive Aufweitung (122) nach distal.

8. Spatel (52) nach einem der Ansprüche 1-7, wobei zumindest das proximale Endprofil (70) des Grundkörpers (60) nach proximal aufgeweitet ist, insbesondere umfassend eine progressive Aufweitung (120) nach proximal.

9. Spatel (52) nach einem der Ansprüche 1-8, wobei der Grundkörper (60) zumindest beim distalen Ende (68) eine atraumatische Aufdickung (126) aufweist, so dass der Grundkörper (60) beim distalen Endprofil (72) eine erhöhte Wandstärke aufweist.

10. Spatel (52) nach einem der Ansprüche 1-9, wobei der Grundkörper (60) zwischen dem distalen Endprofil (72) und dem proximalen Endprofil (70) tailliert ist.

11. Spatel (52) nach einem der Ansprüche 1-10, wobei das distale Endprofil (72) oval, insbesondere eiförmig oder nockenförmig, ist.

12. Spatel (52) nach einem der Ansprüche 1-11, wobei eine Nebenachse (144) des proximalen Endprofils (70) größer als die Hauptachse (142) des distalen Endprofils (72) ist, und wobei die Nebenachse (144) des proximalen Endprofils (70) bei Betrachtung entlang einer Längserstreckung (62) des Grundkörpers (60) mit der Hauptachse (142) des distalen Endprofils (72) in einer Längsmittelebene (98) liegt.

13. Spatel (52) nach einem der Ansprüche 1-12, wobei das distale Endprofil (72) bei einer Projektion auf eine Ebene beim distalen Ende (68), die normal zur Längserstreckung (62) des Grundkörpers (60) ist, eine Fläche einnimmt, die weniger als ein Drittel einer Fläche einer Projektion des proximalen Endprofils (70) auf eine Ebene beim proximalen Ende (66), die normal zur Längserstreckung (62) des Grundkörpers (60) ist, umfasst.

14. Spatel (52) nach einem der Ansprüche 1-13, wobei der Hauptkanal (80) im Grundkörper (60) bei der inneren Mündung (110) des zumindest einen Hilfskanals (82) einen ovalen oder runden Querschnitt (150) aufweist, in den der Hilfskanal (82) mündet.

15. Spatel (52) nach einem der Ansprüche 1-14, wobei der Hauptkanal (80) und der zumindest eine Hilfskanal (82) beim proximalen Endbereich (74) Zugangsöffnungen (132, 134) aufweisen, die zumindest abschnittsweise in einer gemeinsamen Ebene (104) liegen.

16. Spatel (52) nach einem der Ansprüche 1-15, wobei der Hauptkanal (80) im Grundkörper (60) eine Umfangswand (86) aufweist, wobei der zumindest eine Hilfskanal (82) zumindest abschnittsweise zum Hauptkanal (80) benachbart ist und eine Umfangswand (88) aufweist, und wobei sich die Umfangswände (86, 88) des zumindest einen Hilfskanals (82) und des Hauptkanals (80) in einem Überschneidungsbereich (116) zumindest abschnittsweise überschneiden.

17. Spatel (52) nach Anspruch 16, wobei der Überschneidungsbereich (116) entlang der Längserstreckung (62) zwischen dem zumindest einen Hilfskanal (82) und dem Hauptkanal (80) eine Dicke aufweist, die gleich einer Dicke eines benachbarten Abschnitts der Umfangswand (86) des Hauptkanals (80) ist.

18. Spatel (52) nach Anspruch 16 oder 17, wobei ein Übergang (138) zwischen den Umfangswänden (86, 88) des zumindest einen Hilfskanals (82) und des Hauptkanals (80) auskehlungsfrei gestaltet ist.

19. Verfahren zur Herstellung eines medizinischen Instruments in Form eines integral gestalteten Operationslaryngoskop-Spatels (52) nach einem der Ansprüche 1-18, mit den folgenden Schritten:
- Bereitstellung einer Datenverkörperung eines Schafts (56) des Operationslaryngoskop-Spatels (52)
- integrale Fertigung des Schafts (56) in einem additiven Fertigungsverfahren auf Basis eines pulverförmigen metallischen Ausgangsmaterials unter Berücksichtigung der Datenverkörperung, und
- Verbinden des Schafts (56) mit einem Griff (54) am proximalen Ende (66) des Grundkörpers (60) des Schafts (56).
